# EUROPEAN PATENT APPLICATION

(11) **EP 2 047 758 A1**
(43) Date of publication of application: **15.04.2009**
(21) Application number: 07118147.3
(22) Date of filing: 09.10.2007
(51) Int. Cl.: A23L 1/03, C11B 9/00, C07C 33/12, C07D 307/93

(54) **Bicyclic campholenic derivatives**

(71) Applicant: V. Mane Fils, 06620 Bar sur Loup (FR)
(72) Inventor: Mane, Jean, 06130 Grasse (FR); Plessis, Caroline, 06740 Chateauneuf (FR)
(74) Representative: Cabinet Plasseraud

(57) **Abstract**

A compound of general formula (I) 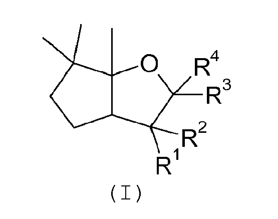 wherein
R¹, R², R³, and R⁴ are, independently, a hydrogen atom or an optionally substituted linear or branched C₁-C₆ alkyl or C₂-C₆ alkenyl group,
provided that when each of R¹, R², and R³ are a hydrogen atom, R⁴ is not a phenyl substituted linear or branched C₁-C₆ alkyl or C₂-C₆ alkenyl group; and
with the proviso that the compound of formula (I) is neither 6,6,6a-trimethyl-hexahydro-cyclopenta[b]furan, 6,6,6a-trimethyl-2-ethyl-hexahydro-cyclopenta[b]furan, nor 6,6,6a-trimethyl-2-propyl-hexahydro-cyclopenta[b]furan and use thereof in a fragrant and/or flavouring composition.

## Description

### [FIELD OF THE INVENTION]

The present invention relates to the field of fragrances and flavours. More particularly, the invention relates to new campholenic derivatives, their method of preparation, and their use in the fields of perfumery and flavouring.

### [BACKGROUND]

Several bicyclic ethers derived from campholenaldehyde, substituted with a phenyl group or a phenyl substituted C₁-C₂ alkyl group, presenting a sweaty, animal, ambery (or woody) odour, have been described in the literature, e.g. in US patent n° 5,276,211.

Bicyclic ethers derived from campholenaldehyde, substituted with an ethyl, phenyl, benzyl, *n*-propyl, or ethylphenyl group in 2-position have been mentioned in an article (Narula, Perfumer & Flavourist, 2003, p. 62 to 73). To the best of our knowledge no other alkyl or alkenyl substituted bicyclic ether derivatives of campholenaldehyde have been described nor claimed to be good odorants. Furthermore, all the processes for preparing bicyclic ethers derived from campholenaldehyde described in the literature lead to several derivatives and the bicyclic ethers are obtained as a mixture of isomers. To the best of our knowledge, no processes allowing the selective synthesis of one bicyclic isomer have been described.

### [PROBLEM TO BE SOLVED]

In view of the drawbacks of the prior art, the Applicant focused on a process to prepare new alkyl or alkenyl substituted bicyclic ethers derived from campholenaldehyde.

The need for new compounds is of great importance for the development of the flavour and fragrance industry, which recently had to face stricter international regulatory requirements about the use of certain materials, as well as environmental concerns and customer demands for improved performance. Providing new fragrant and/or flavouring compounds as well as means of selectively manufacturing such compounds, in particular as a single derivative, is therefore an object of the invention.

In other words, it is an aim of the present invention to provide new flavouring and/or fragrant compounds, as well as a method of manufacturing such compounds.

### [SUMMARY OF THE INVENTION]

The present invention is directed to novel compounds of formula (I) 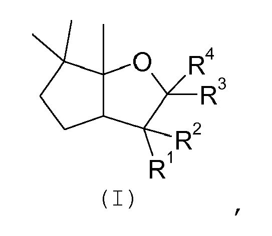 wherein
R¹, R², R³, and R⁴ are, independently, a hydrogen atom or an optionally substituted linear or branched C₁-C₆ alkyl or C₂-C₆ alkenyl group,
provided that when each of R¹, R², and R³ are a hydrogen atom, R⁴ is not a phenyl substituted linear or branched C₁-C₆ alkyl or C₂-C₆ alkenyl group; and
with the proviso that the compound of formula (I) is neither 6,6,6a-trimethyl-hexahydro-cyclopenta[b]furan, 6,6,6a-trimethyl-2-ethyl-hexahydro-cyclopenta[b]furan, nor 6,6,6a-trimethyl-2-propyl-hexahydro-cyclopenta[b]furan.

The invention is also directed to a method of preparation of compounds of formula (I) 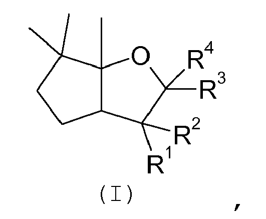 wherein
R¹, R², R³, and R⁴ are, independently, a hydrogen atom or an optionally substituted linear or branched C₁-C₆ alkyl or C₂-C₆ alkenyl group; and to intermediates or side products obtained thereby.

### [DETAILED DESCRIPTION OF THE INVENTION]

As noted above, the invention relates to compounds of formula (I), wherein
R¹, R², R³, and R⁴ are, independently, a hydrogen atom or an optionally substituted linear or branched C₁-C₆ alkyl or C₂-C₆ alkenyl group,
provided that when each of R¹, R², and R³ are a hydrogen atom, R⁴ is not a phenyl substituted linear or branched C₁-C₆ alkyl or C₂-C₆ alkenyl group; and
with the proviso that the compound of formula (I) is neither 6,6,6a-trimethyl-hexahydro-cyclopenta[b]furan, 6,6,6a-trimethyl-2-ethyl-hexahydro-cyclopenta[b]furan, nor 6,6,6a-trimethyl-2-propyl-hexahydro-cyclopenta[b]furan.

Suitable substituents for the alkyl or alkenyl groups comprise hydroxyl, amino groups, aryl groups, such as phenyl, tolyl (e.g. *p*-tolyl or *o*-tolyl), *p*-methoxy-phenyl, heterocycloalkyl groups, such as pyrolidinyl, tetrahydrofuranyl, and tetrahydro-pyranyl, heteroaryl groups such as pyridinyl, furanyl, and pyranyl, O-alkyl groups, such as methoxy, ethoxy, *i*-propoxy, phenoxy.

Preferred compounds of formula (I) are those, wherein R¹, R², R³, and R⁴ are, independently, a hydrogen atom or an optionally substituted methyl, ethyl, *n*-propyl, *i*-propyl, *n-*butyl, *i*-butyl, *s*-butyl, *t*-butyl, *n*-pentyl, *i*-pentyl, *t*-amyl, 1-ethyl-propyl, 1,2-dimethyl-propyl, 2,2-dimethyl-propyl, *n-*hexyl, 1-methyl-pentyl group, 2-methyl-pentyl group, 3-methyl-pentyl group, 4-methyl-pentyl group, 1,1-dimethyl-butyl, 1,2-dimethyl-butyl, 1,3-dimethyl-butyl, 2,2-dimethyl-butyl, 3,3-dimethyl-butyl, 1-ethyl-butyl, 2-ethyl-butyl, 1,1,2-trimethyl-propyl, 1,2,2-trimethyl-propyl, 1-ethyl-1-methyl-propyl or 1-ethyl-2-methyl-propyl, or an optionally substituted vinyl, 1-propenyl, 1-*i*-propenyl, 2-propenyl, 2-methyl-1-propenyl, 1-butenyl, 2-butenyl, 3-butenyl, 1-pentenyl, 2-pentenyl, 1-hexenyl, 2-hexenyl, 3-hexenyl, 2,4-pentadienyl group, with, as mentioned above, the proviso that when each of R¹, R², and R³ are a hydrogen atom, R⁴ is not a phenyl substituted linear or branched C₁-C₆ alkyl or C₂-C₆ alkenyl group.

Particularly preferred compounds of formula (I) are those, wherein R¹, R², and R³, are each a hydrogen atom and R⁴ is an optionally substituted linear or branched C₁-C₆ alkyl or C₂-C₆ alkenyl group, such as an optionally substituted methyl, ethyl, *n*-propyl, *i*-propyl, *n*-butyl, *i*-butyl, *s*-butyl, *t*-butyl, *n*-pentyl, *i*-pentyl, *t*-amyl, 1-ethyl-propyl, 1,2-dimethyl-propyl, 2,2-dimethyl-propyl, *n*-hexyl, 1-methyl-pentyl group, 2-methyl-pentyl group, 3-methyl-pentyl group, 4-methyl-pentyl group, 1,1-dimethyl-butyl, 1,2-dimethyl-butyl, 1,3-dimethyl-butyl, 2,2-dimethyl-butyl, 3,3-dimethyl-butyl, 1-ethyl-butyl, 2-ethyl-butyl, 1,1,2-trimethyl-propyl, 1,2,2-trimethyl-propyl, 1-ethyl-1-methyl-propyl or 1-ethyl-2-methyl-propyl, or an optionally substituted vinyl, 1-propenyl, 1-*i*-propenyl, 2-propenyl, 2-methyl-1-propenyl, 1-butenyl, 2-butenyl, 3-butenyl, 1-pentenyl, 2-pentenyl, 1-hexenyl, 2-hexenyl, 3-hexenyl, 2,4-pentadienyl group, with the proviso that the optional substituent is not a phenyl group.

The invention is also directed to a method of preparing a compound of general formula (I) 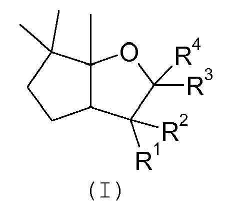 wherein
R¹, R², R³, and R⁴ are, independently, a hydrogen atom or an optionally substituted linear or branched C₁-C₆ alkyl or C₂-C₆ alkenyl group,
the method comprising reacting a compound of formula (III) 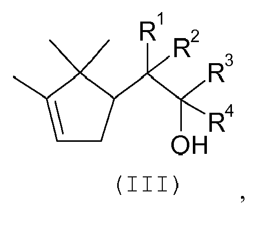 wherein
R¹, R², R³, and R⁴ are defined as in respect of formula (I),
with phosphoric acid, preferably concentrated phosphoric acid, in an organic solvent so as to obtain a mixture comprising a compound of formula (I) and a compound of formula (II) 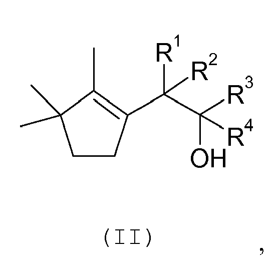 wherein
R¹, R², R³, and R⁴ are defined as in respect of formula (I).

Suitable substituents for the alkyl or alkenyl groups are those listed above in respect of compounds of formula (I).

Preferred compounds of formula (I) obtained according to the method of the invention are those, wherein R¹, R², R³, and R⁴ are, independently, a hydrogen atom or an optionally substituted methyl, ethyl, *n*-propyl, *i*-propyl, *n*-butyl, *i-*butyl, *s*-butyl, *t*-butyl, *n*-pentyl, *i*-pentyl, *t*-amyl, 1-ethyl-propyl, 1,2-dimethyl-propyl, 2,2-dimethyl-propyl, *n*-hexyl, 1-methyl-pentyl group, 2-methyl-pentyl group, 3-methyl-pentyl group, 4-methyl-pentyl group, 1,1-dimethyl-butyl, 1,2-dimethyl-butyl, 1,3-dimethyl-butyl, 2,2-dimethyl-butyl, 3,3-dimethyl-butyl, 1-ethyl-butyl, 2-ethyl-butyl, 1,1,2-trimethyl-propyl, 1,2,2-trimethyl-propyl, 1-ethyl-1-methyl-propyl or 1-ethyl-2-methyl-propyl, or an optionally substituted vinyl, 1-propenyl, 1-*i*-propenyl, 2-propenyl, 2-methyl-1-propenyl, 1-butenyl, 2-butenyl, 3-butenyl, 1-pentenyl, 2-pentenyl, 1-hexenyl, 2-hexenyl, 3-hexenyl, 2,4-pentadienyl group.

Particularly preferred compounds of formula (I) obtained according to the method of the invention are those, wherein R¹, R², and R³, are each a hydrogen atom and R⁴ is an optionally substituted linear or branched C₁-C₆ alkyl or C₂-C₆ alkenyl group, such as methyl, ethyl, *n*-propyl, *i*-propyl, *n*-butyl, *i-*butyl, *s*-butyl, *t*-butyl, *n*-pentyl, *i*-pentyl, *t*-amyl, 1-ethyl-propyl, 1,2-dimethyl-propyl, 2,2-dimethyl-propyl, *n*-hexyl, 1-methyl-pentyl group, 2-methyl-pentyl group, 3-methyl-pentyl group, 4-methyl-pentyl group, 1,1-dimethyl-butyl, 1,2-dimethyl-butyl, 1,3-dimethyl-butyl, 2,2-dimethyl-butyl, 3,3-dimethyl-butyl, 1-ethyl-butyl, 2-ethyl-butyl, 1,1,2-trimethyl-propyl, 1,2,2-trimethyl-propyl, 1-ethyl-1-methyl-propyl or 1-ethyl-2-methyl-propyl, or an optionally substituted vinyl, 1-propenyl, 1-*i*-propenyl, 2-propenyl, 2-methyl-1-propenyl, 1-butenyl, 2-butenyl, 3-butenyl, 1-pentenyl, 2-pentenyl, 1-hexenyl, 2-hexenyl, 3-hexenyl, 2,4-pentadienyl group. Preferably, the optional substituent is not a phenyl group.

The reaction of the compound of formula (III) with phosphoric acid is advantageously carried out at a temperature of room temperature to refluxing solvent, preferably about 50°C to 80°C and even more preferably about 70°C to 80°C

The organic solvent may be selected from the group comprising toluene, xylene, trimethylbenzene, cyclohexane, and methylcyclohexane. According to a preferred embodiment, the organic solvent is toluene.

The ratio of compound (I) to compound (II) depends among others on the reaction temperature, e.g. using toluene as organic solvent the ratio is of about 20:80 at a temperature of 80°C and 50:50 at 110°C (reflux).

The phosphoric acid is advantageously used in an amount of 1 to 10% by weight of compound (III), preferably 2 to 5% by weight of compound (III). In a particularly preferred embodiment the phosphoric acid is concentrated phosphoric acid.

The compound of formula (II) may be separated from the compound of formula (I) by any suitable technique known in the art, such as distillation, HPLC, or column chromatography on silica gel.

The main advantage of the method of the invention is that the secondary product, i.e. the compound of formula (II), may easily be transformed into the corresponding compound of formula (I). The transformation of compound (II) into compound (II) is carried out by reacting compound (II) with trifluoromethanesulphonic acid in an organic solvent.

According to an advantageous variant of the method of the invention, the method of the invention therefore further comprises reacting the compound of formula (II) with trifluoromethanesulphonic acid (triflic acid). It is thus possible to selectively synthesize compounds of formula (I).

The reaction with trifluoromethanesulphonic acid is advantageously carried out at a temperature of about room temperature to refluxing solvent, preferably at a temperature of about 50 to 80°C, even more preferably of about 70 to 80°C.

According to an advantageous variant of the method of the invention, the transformation may either be carried out as a one-pot reaction, i.e. the trifluoromethanesulphonic acid is directly added to the reaction mixture obtained from reacting a compound of formula (III) with phosphoric acid as described above, or after separating the compound of formula (II) from the compound of formula (I) (two-pot reaction).

In the case where the transformation of compound (I) into compound (II) is carried out as a one-pot reaction, the conditions in respect of temperature and solvent are those set out above.

The amount of trifluoromethanesulphonic acid used is advantageously of about 0.5 to 5% by weight of compound (III), preferably about 0.7 to 3% by weight of compound (III), and even more preferably of about 1 to 2% by weight of compound (III).

In the case where the compound of formula (II) is separated from the reaction mixture prior to its transformation into the corresponding compound of formula (I) (two-pot reaction), the separation may be carried out by any suitable technique known in the art, such as distillation, HPLC, or column chromatography on silica gel.

The organic solvent used in the two-pot reaction is selected from the group comprising toluene, xylene, trimethylbenzene, cyclohexane, and methylcyclohexane. According to a preferred embodiment, the organic solvent is toluene.

In the case of a two-pot reaction, the transformation is advantageously carried out at a temperature of about room temperature to refluxing solvent, preferably at a temperature of about 50 to 80°C, even more preferably of about 70 to 80°C.

In the two-pot reaction, the trifluoromethanesulphonic acid is advantageously used in an amount of about 0.5 to 5% by weight of compound (II), preferably about 0.7 to 3% by weight of compound (II), and even more preferably of about 1 to 2% by weight of compound (II).

The starting compound of the method of the invention, i.e. the compound of formula (III) may easily be prepared by condensation of an appropriately substituted organometallic reagent (e.g. a Grignard reagent) with campholenaldehyde derived compounds (prepared according to known procedures from the person of the art).

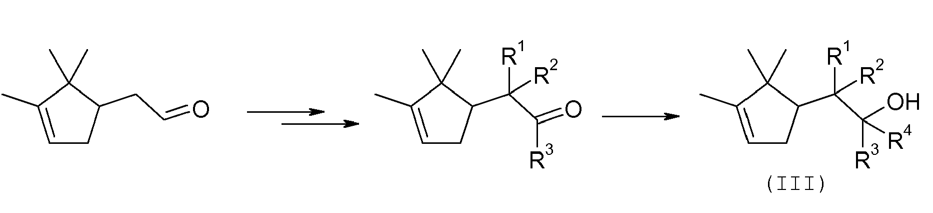

The invention is also directed to compounds of formula (II), wherein R¹, R², R³, and R⁴ are, independently, a hydrogen atom or an optionally substituted linear or branched C₁-C₆ alkyl or C₂-C₆ alkenyl group, provided that when each of R¹, R², and R³ are a hydrogen atom, R⁴ is not a phenyl substituted linear or branched C₁-C₆ alkyl or C₂-C₆ alkenyl group; and with the proviso that the compound of formula (II) is neither 2-(2,3,3-trimethyl-cyclopent-1-enyl)-ethanol, 1-(2,3,3-trimethyl-cyclopent-1-enyl)-butan-2-ol, nor 1-(2,3,3-trimethyl-cyclopent-1-enyl)-pent-3-en-2-ol.

A further object of the invention is a fragrant and/or flavouring composition containing at least one compound of formula (I) or of formula (II) 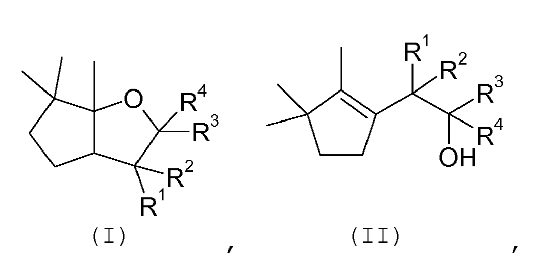 wherein R¹, R², R³, and R⁴ are, independently, a hydrogen atom or an optionally substituted linear or branched C₁-C₆ alkyl or C₂-C₆ alkenyl group, provided that when each of R¹, R², and R³ are a hydrogen atom, R⁴ is not a phenyl substituted linear or branched C₁-C₆ alkyl or C₂-C₆ alkenyl group; with the proviso that the compound of formula (I) is neither 6,6,6a-trimethyl-hexahydro-cyclopenta[b]furan, 6,6,6a-trimethyl-2-ethyl-hexahydro-cyclopenta[b]furan, nor 6,6,6a-trimethyl-2-propyl-hexahydro-cyclopenta[b]furan and that the compound of formula (II) is neither 2-(2,3,3-trimethyl-cyclopent-1-enyl)-ethanol, 1-(2,3,3-trimethyl-cyclopent-1-enyl)-butan-2-ol, nor 1-(2,3,3-trimethyl-cyclopent-1-enyl)-pent-3-en-2-ol.

This invention includes any composition comprising one or more isomers of a compound of formula (I) and/or of a compound of formula (II). According to a preferred embodiment, this invention relates to a composition comprising at least two or three isomers of a compound of formula (I) and/or of a compound of formula (II).

The fragrant and/or flavouring composition of the invention may further comprise other perfuming or flavouring ingredients, solvents, additives or fixatives, commonly used and that the man skilled in the art is able to choose in regard of the desired effect and the nature of the product to perfume or flavour.

A further object of the invention is the use of a compound of formula (I) or of formula (II), wherein R¹, R², R³, and R⁴ are, independently, a hydrogen atom or an optionally substituted linear or branched C₁-C₆ alkyl or C₂-C₆ alkenyl group, or of a fragrant composition as described above, in the perfumery field, for the preparation of perfumed bases and concentrates, fragrances, perfumes; topic compositions; cosmetic compositions, such as face and body creams, cleansers, facial treatments, talc powders, hair oils, shampoos, hair lotions, bath oils and salts, shower and bath gels, soaps, body anti-perspirants and deodorizers, pre-shave, shaving and post-shave creams and lotions, creams, toothpastes, mouth baths, pomades; and cleaning products, such as softeners, detergents, air deodorizers and household cleaning supplies.

The invention is also directed to the use of a compound of formula (I) or of formula (II), wherein R¹, R², R³, and R⁴ are, independently, a hydrogen atom or an optionally substituted linear or branched C₁-C₆ alkyl or C₂-C₆ alkenyl group, or of a flavouring composition as described above, as flavouring agent for the preparation of foodstuffs, drinks, and tobacco. The foodstuffs and drinks are preferably selected from the group consisting of dairy products, ice creams, soups, sauces, dips, dishes, meat products, culinary assistances, salted biscuits, snacks, soft drinks, beers, wines and spirits.

The invention is also directed to the use of a compound of formula (I) or of formula (II), wherein R¹, R², R³, and R⁴ are, independently, a hydrogen atom or an optionally substituted linear or branched C₁-C₆ alkyl or C₂-C₆ alkenyl group, or of a fragrant and/or flavouring composition as described above, as masking agent of odours and/or flavours, e.g. in pharmaceutical, cosmetic or food compositions.

The invention also provides the use of a compound of formula (I) or of formula (II), wherein R¹, R², R³, and R⁴ are, independently, a hydrogen atom or an optionally substituted linear or branched C₁-C₆ alkyl or C₂-C₆ alkenyl group, or of a fragrant or flavouring composition as described above, in combination with other perfumed or flavoured ingredients, solvents or additives or fixatives.

The compounds of the invention may be used in a concentration comprised in a range from 0.001% to 99% in weight, preferably from 0.1% to 50% in weight, more preferably from 0.1% to 30% in weight. It is known by the man skilled in the art that these values depend on the nature of the composition/article to be perfumed and/or flavoured, the desired intensity of the perfume and/or flavour, and of the nature of the other ingredients present in said composition or article. According to a preferred embodiment of the invention, compounds are used in an olfactory effective amount.

### [DEFINITIONS]

The terms "fragrance" and "fragrant", as used herein, are used interchangeably whenever a compound or a mixture of compounds is referred to, which is intended to pleasantly stimulate the sense of smell.

The terms "flavour" and "flavouring", as used herein, are used interchangeably whenever a compound or a mixture of compounds is referred to, which is intended to stimulate the sense of taste and smell. Also in the meaning of the invention, the term "flavouring" relates to the flavouring of any liquid or solid, human or animal, in particular of drinks, dairy products, ice creams, soups, sauces, dips, dishes, meat products, culinary assistances, salted biscuits or snacks. It also means the flavouring of beers, wines and tobaccos.

The term "olfactory effective amount", as used herein, means a level or amount of fragrant/flavouring compound present in a material at which the incorporated compound exhibits a sensory effect.

By the term "masking" is meant reducing or eliminating malodour or bad flavour perception generated by one or more molecules entering in the composition of a product.

The term "alkyl" or "alkyl group", in the present invention, means any linear or branched saturated hydrocarbon chain, having preferably 1, 2, 3, 4, 5 or 6 carbon atoms and herein referred to as C₁-C₆ alkyl group, such as for example methyl, ethyl, propyl, isopropyl, butyl, *t*-butyl, pentyl, or hexyl.

The term "alkenyl" or "alkenyl group", in the present invention, means any linear or branched mono or poly unsaturated hydrocarbon chain, having preferably 2, 3, 4, 5 or 6 carbon atoms and herein referred to as C₂-C₆ alkenyl group, such as for example ethenyl (vinyl), propenyl, butenyl, pentenyl, or hexenyl.

The term "isomer", in the present invention, means molecules having the same chemical formula, which means same number and types of atoms, but in which the atoms are arranged differently. The term "isomer" includes structural isomers, geometric isomers, optical isomers and stereoisomers.

The term "room temperature", as used herein, means a temperature of about 18°C to about 25°C.

The present invention will be better understood with reference to the following examples. These examples are intended to representative of specific embodiments of the invention, and are not intended as limiting the scope of the invention.

### Example 1: Preparation of 2-isobutyl-6,6,6a-trimethyl-hexahydro-cyclopenta[b]furan and of 4-methyl-1-(2,3,3-trimethyl-cyclopent-1-enyl)-pentan-2-ol

A solution of 4-ethyl-1-(2,2,3-trimethyl-cyclopent-3-enyl)-pentan-2-ol (obtained from campholenaldehyde and 2-methyl-propylmagnesium bromide) in toluene with 3% weight of concentrated phosphoric acid is heated under reflux until completion of the reaction. After cooling down, the reaction mixture is washed with a saturated aqueous sodium bicarbonate solution and with brine. The organic layer is then dried over magnesium sulphate, filtered and the solvents are evaporated. The single compounds are obtained by distillation of a (50:50) mixture of 2-isobutyl-6,6,6a-trimethyl-hexahydro-cyclopenta-[b]furan and of 4-methyl-1-(2,3,3-trimethyl-cyclopent-1-enyl)-pentan-2-ol.

### 2-Isobutyl-6,6,6a-trimethyl-hexahydro-cyclopenta[b]furan

Obtained as a (87:13) ratio of isomers.
Minty, blackcurrant (blackcurrant buds aspects), earthy odour with sweat aspects, reminiscent of bucchu essential oil.
Bp = 35°C/0.4 torr - 94°C/6 torr
Main isomer:
**¹H-NMR** (200MHz, CDCl₃) : δ 0.81 (t, 3H), 0.88 (d, 3H, *J* = 6.4Hz), 0.89 (d, 3H, *J* = 6.4Hz), 0.99 (s, 3H), 1.07 (s, 3H), 1.05-1.4 (m, 3H), 1.4-1.78 (m, 5H), 1.78-2.1 (m, 1H), 2.25-2.44 (m, 1H), 3.89 (dt, 1H, *J* = 6.3Hz, *J* = 12.0Hz).
**¹³C-NMR** (50MHz, CDCl₃) : δ 20.81, 22.17, 22.83, 23.10, 25.37, 25.83, 29.44, 40.41, 40.97, 46.39, 46.46, 47.69, 78.49, 94.26.
Minor isomer:
**¹H-NMR** (200MHz, CDCl₃, selected data) : δ 0.98 (s, 3H), 1.05 (s, 3H).
**¹³C-NMR** (50MHz, CDCl₃) : δ 18.15, 22.16, 22.93, 23.23, 24.48, 25.73, 31.25, 38.43, 42.07, 44.72, 45.20, 47.94, 75.98, 94.06.
**IR** (film, cm⁻¹) : 896w, 1010m, 1073m, 1092m, 1118m, 1368m, 1383m, 1444m, 1465m, 2870s, 2954s.

### 4-Methyl-1-(2,3,3-trimethyl-cyclopent-1-enyl)-pentan-2-ol

Light woody odour.
Bp = 65°C/0.4 torr
**¹H-NMR** (200MHz, CDCl₃) : δ 0.89 (d, 1H, *J* = 3.0Hz), 0.93 (d, 1H, *J* = 3.1Hz), 0.97 (s, 3H), 0.99 (s, 3H), 1.20 (ddd, 1H, *J* = 4.1Hz, *J* = 8.8Hz, *J* = 13.7Hz), 1.41 (ddd, 1H, *J* = 5.3Hz, *J* = 8.8Hz, *J* = 16.9Hz), 1.53 (t, 3H, *J* = 1.9Hz), 1.57-1.68 (m, 2H), 1.68-1.95 (m, 1H), 2.0-2.4 (m, 4H), 3.76 (qd, 1H, *J* = 4.4Hz, *J* = 12.9Hz).
**¹³C-NMR** (50MHz, CDCl₃) : δ 9.62, 22.11, 23.49, 24.77, 26.16, 26.78, 32.92, 37.86, 38.86, 46.29, 47.0, 67.82, 129.93, 143.21.
**IR** (film, cm⁻¹) : 1019w, 1074m, 1360m, 1383w, 1464m, 2843m, 2867m, 2932s, 2955s, 3360m br.
**MS** (EI, intensity (%)) : 210(M+, 3), 195(1), 177(4), 109(100), 69(12), 43(12), 41(12).

### Example 2: Preparation of 2-isobutyl-6,6,6a-trimethyl-hexahydro-cyclopenta[b]furan from 4-methyl-1-(2,3,3-trimethyl-cyclopent-1-enyl)-pentan-2-ol

A solution of 4-methyl-1-(2,3,3-trimethyl-cyclopent-1-enyl)-pentan-2-ol in toluene is heated at 80°C and 1% weight of triflic acid is added. After completion of the reaction, the reaction mixture is cooled down and washed with a saturated aqueous sodium bicarbonate solution and with brine. The organic layer is then dried over magnesium sulphate, filtered and the solvents are evaporated. The crude 2-isobutyl-6,6,6a-trimethyl-hexahydro-cyclopenta[b]furan is purified by distillation.

### Example 3: "One pot" preparation of 2-isobutyl-6,6,6a-trimethyl-hexahydro-cyclopenta[b]furan

A solution of 4-methyl-1-(2,2,3-trimethyl-cyclopent-3-enyl)-pentan-2-ol (obtained from campholenaldehyde and 2-methyl-propylmagnesium bromide) in toluene with 3% weight of phosphoric acid is heated at 80°C until 4-methyl-1-(2,2,3-trimethyl-cyclopent-3-enyl)-pentan-2-ol is totally converted into 4-methyl-1-(2,3,3-trimethyl-cyclopent-1-enyl)-pentan-2-ol. After cooling down to room temperature, 1% weight of triflic acid is added and the reaction mixture is further stirred until 4-methyl-1-(2,3,3-trimethyl-cyclopent-1-enyl)-pentan-2-ol is converted into 2-isobutyl-6,6,6a-trimethyl-hexahydro-cyclopenta[b]furan.
After completion of the reaction, the work-up is carried out according to example 2.

### Example 4: Preparation of 1-(2,3,3-trimethyl-cyclopent-1-enyl)-but-3-en-2-ol

A solution of 1-(2,2,3-trimethyl-cyclopent-3-enyl)-but-3-en-2-ol (obtained from campholenaldehyde and vinylmagnesium bromide) in toluene with 3% weight of concentrated phosphoric acid is heated at 80°C until completion of the reaction. After cooling down, the reaction mixture is washed with a saturated aqueous sodium bicarbonate solution and with brine. The organic layer is then dried over magnesium sulphate, filtered and the solvents are evaporated.

The crude product consists in a (20:80) mixture of 6,6,6a-trimethyl-2-vinyl-hexahydro-cyclopenta[b]furan and 1-(2,3,3-trimethyl-cyclopent-1-enyl)-but-3-en-2-ol.

### 1-(2,3,3-Trimethyl-cyclopent-1-enyl)-but-3-en-2-ol

**¹H-NMR** (200MHz, CDCl₃) : δ 0.97 (s, 3H), 0.99 (s, 3H), 1.0-1.4 (m, 1H), 1.4-1.6 (m, 3H), 1.6-2 (m, 2H), 2.0-2.4 (m, 3H), 4.2 (m, 1H), 5.09 (td, 1H, *J* = 1.5Hz, *J* = 10.4Hz), 5.24 (td, 1H, *J* = 1.5Hz, *J* = 17.2Hz), 5.87 (ddd, 1H, *J* = 5.8Hz, *J* = 10.4Hz, *J* = 17.2Hz).

**¹³C-NMR** (50MHz, CDCl₃) : δ 9.63, 26.22, 26.65, 32.96, 37.15, 38.84, 46.95, 71.09, 114.26, 137.81, 140.66, 143.36.

**MS** (EI, intensity (%)) : 180(M+, 4), 165(12), 147 (11), 124 (17), 123 (68), 109(92), 95 (18), 93(20), 91 (24), 81(100), 79 (23), 77(21), 67(38), 57(36), 55(33), 43(15), 41(28), 39(15).

### Example 5: Preparation of 6,6,6a-trimethyl-2-vinyl-hexahydro-cyclopenta[b]furan

1-(2,3,3-Trimethyl-cyclopent-1-enyl)-but-3-en-2-ol, obtained in example 4, is converted into 6,6,6a-trimethyl-2-vinyl-hexahydro-cyclopenta[b]furan according to example 2.

### 6,6,6a-Trimethyl-2-vinyl-hexahydro-cyclopenta[b]furan Obtained in a (80:20) ratio of isomers.

Woody, camphoraceous.
Bp = 65°C/5 torr
Major isomer:
**¹H-NMR** (200MHz, CDCl₃) : δ 0.83 (s, 3H), 1.02 (s, 3H), 1.08-1.47 (m, 2H), 1.12 (s, 3H), 1.47-1.75 (m, 1H), 1.75-2.5 (m, 4H), 4.28 (dd, 1H, *J* = 7.0Hz, *J* = 16.6Hz), 5.05 (ddd, 1H, *J* = 0.8Hz, *J* = 1.8Hz, *J* = 10.1Hz), 5.21 (ddd, 1H, *J* = 0.9Hz, *J* = 1.8Hz, *J* = 17.1Hz), 5.79 (ddd, 1H, *J* = 7.2Hz, *J* = 10.1Hz, *J* = 17.2Hz).
**¹³C-NMR** (50MHz, CDCl₃) : δ 20.36, 22.12, 25.38, 29.09, 40.13, 41.08, 46.39, 47.84, 81.44, 95.61, 115.32, 140.55. Minor isomer (selected data):
**¹H-NMR** (200MHz, CDCl₃) : δ 0.89 (s, 3H), 1.03 (s, 3H), 1.1 (s, 3H), 2.5-2.7 (m, 1H), 4.3 (m, 1H), 5.04 (ddd, 1H, *J* = 1.1Hz, *J* = 1.8Hz, *J* = 10.3Hz), 5.23 (ddd, 1H, *J* = 1.3Hz, *J* = 1.8Hz, *J* = 17.2Hz), 5.87 (ddd, 1H, *J* = 6.6Hz, *J* = 10.4Hz, *J* = 17Hz).
**¹³C-NMR** (50MHz, CDCl₃) : δ 18.25, 22.23, 24.63, 30.84, 38.4, 42.11, 44.86, 48.15, 78.71, 95.16, 114.44, 139.42.

### Example 6: "One pot" preparation of 6,6,6a-trimethyl-2-vinyl-hexahydro-cyclopenta[b]furan

1-(2,2,3-Trimethyl-cyclopent-3-enyl)-but-3-en-2-ol (obtained from campholenaldehyde and vinylmagnesium bromide) was prepared according to example 3, using 5% weight of phosphoric acid and 2% weight of triflic acid.

### Example 7: Preparation of 2-ethyl-6,6,6a-trimethyl-hexahydro-cyclopenta[b]furan and of 1-(2,3,3-Trimethyl-cyclopent-1-enyl)-butan-2-ol

The single compounds were obtained by distillation of a (50:50) mixture prepared, via ethylmagnesium bromide with campholenaldehyde, according to example 1.

### 2-Ethyl-6,6,6a-trimethyl-hexahydro-cyclopenta[b]furan

2 isomers in a (85:15) ratio, having an intense camphoraceous, green, animalic and cassis-like odour, reminiscent of bucchu essential oil.
Bp = 65°C/5 torr
Major isomer:
**¹H-NMR** (500MHz, CDCl₃) : δ 0.82 (s, 3H), 0.87 (t, 3H, *J* = 7.5Hz), 1.0 (s, 3H), 1.09 (s, 3H), 1.17-1.25 (m, 1H), 1.32 (dd, 1H, *J* = 8.2Hz, *J* = 12.1Hz), 1.40 (td, 1H, *J* = 7.4Hz, *J* = 13.2Hz), 1.55-1.78 (m, 4H), 1.97 (m, 1H), 2.37 (m, 1H), 3.79 (td, 1H, J=5.3Hz, J=12.4Hz).
**¹³C-NMR** (50MHz, CDCl₃) : δ 10.09, 20.61, 22.15, 25.39, 29.41, 29.46, 39.69, 40.35, 46.4, 47.68, 81.46, 94.56.
Minor isomer:
**¹H-NMR** (500MHz, CDCl₃, selected data) : δ 0.89 (t, 1H, *J* = 7.6Hz), 2.29-2.33 (m, 1H).
**¹³C-NMR** (50MHz, CDCl₃) : δ 10.09, 18.17, 22.15, 24.52, 28.50, 31.21, 38.50, 40.80, 44.82, 47.84, 78.79, 94.38.
**IR** (film, cm⁻¹) : 985m, 1079m, 1117m, 1368m, 1383m, 1453m, 1465m, 2870s, 2959s.
**MS** (EI, intensity (%)) : 182 (21), 167 (2), 153 (2), 126 (9), 125(100), 112(10), 111(6), 109(8), 83(9), 81(5), 71(8), 69(8), 55(18), 43(37), 41(20).

### 1-(2,3,3-Trimethyl-cyclopent-1-enyl)-butan-2-ol

Woody, camphoraceous odour, with mouldy aspects.
Bp = 91-92°C/5 torr
**¹H-NMR** (200MHz, CDCl₃) : δ 0.93-1.0 (m, 3H), 1.35-1.51 (m, 2H), 1.53 (s, 3H), 1.55-1.68 (m, 3H), 2.10-2.23 (m, 2H), 2.23-2.32 (m, 1H), 3.57-3.65 (m, 1H).
**¹³C-NMR** (50MHz, CDCl₃) : δ 9.59, 10.06, 26.18, 26.74, 29.72, 32.90, 36.88, 38.85, 46.97, 71.15, 129.95, 143.07.
**IR** (film, cm⁻¹) : 971m, 1114m, 1359w, 1460m, 2865m, 3031s, 3058s, 3383m br.
**MS** (EI, intensity (%)): 182(M+, 8), 167(9), 149(8), 109(100), 59(11).

### Example 8: Preparation of 2-ethyl-6,6,6a-trimethyl-hexahydro-cyclopenta[b]furan from 1-(2,3,3-trimethyl-cyclopent-1-enyl)-butan-2-ol

1-(2,3,3-Trimethyl-cyclopent-1-enyl)-butan-2-ol was converted into 2-ethyl-6,6,6a-trimethyl-hexahydro-cyclopenta[b]furan according to example 2.

### Example 9: Preparation of 2-sec-butyl-6,6,6a-trimethyl-hexahydro-cyclopenta[b]furan

2-*sec*-Butyl-6,6,6a-trimethyl-hexahydro-cyclopenta[b]furan was prepared from 3-methyl-1-(2,2,3-trimethyl-cyclopent-3-enyl)-pentan-2-ol (obtained from campholenaldehyde and 1-methyl-propylmagnesiumbromide) according to example 3, using 3% weight of phosphoric acid at 100°C in toluene, then 1% weight of triflic acid at 80°C.
It was obtained as a 45:45:5:5 ratio of isomers.
Blackcurrant, blackcurrant buds effect, camphoraceous, citral.
Bp = 96-98°C/ 8 torr
Major isomers:
**¹H-NMR** (200MHz, CDCl₃) : δ 0.76-0.9 (m, 3H), 0.82 (s, 3H), 0.90 (d, 1H, *J* = 6.7Hz), 0.99 (s, 3H), 1.05 & 1.06 (s, 3H), 1.1-1.35 (m, 2H), 1.35-1.65 (m, 4H), 1.65-1.85 (m, 2H), 1.85-2.1 (m, 1H), 2.27-2.41 (m, 1H), 3.66 (td, 1H, *J* = 5.5Hz, *J* = 11.1Hz).
**¹³C-NMR** (50MHz, CDCl₃) : δ 11.55 & 11.64, 14.13 & 15.62, 20.32, 22.16, 25.06 & 26.73, 25.41, 29.49, 36.68 & 37.09, 39.79, 40.49, 47.43 & 47.48, 84.12 & 84.42, 93.91 & 94.07.
Minor isomers:
**¹H-NMR** (200MHz, CDCl₃, selected data) : δ 1.02 & 1.03 (s, 3H), 3.47 (m, 1H).
**¹³C-NMR** (50MHz, CDCl₃, selected data) : δ 81.09 & 81.5.
**IR** (film, cm⁻¹) : 896w, 1009m, 1077s, 1118m, 1370m, 1382m, 1458m, 1464m, 2873s, 2960s.

### Example 10: Preparation of 2-butyl-6,6,6a-trimethyl-hexahydro-cyclopenta[b]furan

2-Butyl-6,6,6a-trimethyl-hexahydro-cyclopenta[b]furan was prepared from 1-(2,2,3-Trimethyl-cyclopent-3-enyl)-hexan-2-ol 1-(2,3,3-Trimethyl-cyclopent-1-enyl)-hexan-2-ol (obtained from campholenaldehyde and butyllithium) according to example 3.

It was obtained as a 90:10 ratio of isomers.
Blackcurrant, blackcurrant buds, red berries.
Bp = 93-94°C / 7 torr
Major isomer:
**¹H-NMR** (200MHz, CDCl₃) : δ 0.81 (s, 3H), 0.82-0.94 (m, 3H), 1.0 (s, 3H), 1.08 (s, 3H), 1.10-1.45 (m, 6H), 1.5-1.8 (m, 4H), 1.8-2.1 (m, 2H), 2.3-2.45 (m, 1H), 3.82 (m, *J* = 5.9Hz, *J* = 12.2Hz).
**¹³C-NMR** (50MHz, CDCl₃) : δ 14.05, 20.68, 22.146, 22.88, 25.376, 28.32, 29.40, 36.72, 40.35 (2C), 46.38, 47.71, 80.27, 94.42.
Minor isomer:
**¹H-NMR** (200MHz, CDCl₃, selected data) : δ 3.55-3.75 (m, 1H).
**¹³C-NMR** (50MHz, CDCl₃, selected data) : δ 46.98, 47.90, 77.61, 94.23.
**IR** (film, cm⁻¹) : 899w, 1010m, 1074m, 1090m, 1117m, 1368m, 1382m, 1458m, 1464m, 2868s, 2936s, 2956s.

### Example 11: Fragrance composition containing 2-ethyl-6,6,6a-trimethyl-hexahydro-cyclopenta[b]furan

A blackcurrant type accord was prepared as described from the following ingredients:

| **Ingredient** | **Accord 1** | **Accord 2** | **Accord 3** | **Accord 4** |
|---|---|---|---|---|
| DIMETHYLOCTANOL | 15 | 15 | 15 | 15 |
| DYNASCONE® 10 | 20 | 20 | 20 | 20 |
| JASMOLACTONE | 10 | 10 | 10 | 10 |
| METHYL DIHYDRO JASMONATE | 120 | 120 | 120 | 120 |
| NEROL | 20 | 20 | 20 | 20 |
| ROSE OXIDE 10% DPG | 40 | 40 | 40 | 40 |
| STRAWBERRY FURANONE 30% Alc. | 15 | 15 | 15 | 15 |
| TRIPLAL® | 15 | 15 | 15 | 15 |
| VELOUTONE® | 20 | 20 | 20 | 20 |
| DIPROPYLENE GLYCOL | 725 | 715 | 675 | 625 |
| 2-ethyl-6,6,6a-trimethyl-hexahydrocyclopenta[b]furan | - | 10 | 50 | 100 |

These 4 compositions, containing different amount of the bicyclic ether, were used in a shower gel at usual dilution, known from the person of the art, and the samples containing the compound of formula (I) showed a greener metallic note, with earthy aspects.

### Example 12: Fragrance composition containing 2-ethyl-6,6,6a-trimethyl-hexahydro-cyclopenta[b]furan or 2-isobutyl-6,6,6a-trimethyl-hexahydro-cyclopenta[b]furan

To a blackcurrant type accord, prepared as described from the following ingredients (column 2), is added 2-ethyl-6,6,6a-trimethyl-hexahydro-cyclopenta[b]furan (column 3) or 2-isobutyl-6,6,6a-trimethyl-hexahydro-cyclopenta[b]furan (column 4). The 2 new compositions are compared to the primary one to evaluate the impact of the added ingredient.

| **Ingredient** | **Accord 1** | **Accord 2** | **Accord 3** | **Accord 4** | **Accord 5** |
|---|---|---|---|---|---|
| TEREBENTHINE ESS | 15 | 15 | 15 | 15 | 15 |
| PINENE BETA | 100 | 100 | 100 | 100 | 100 |
| ORANGE TERPENES BRESIL | 30 | 30 | 30 | 30 | 30 |
| HEXYLE ACETATE | 3 | 3 | 3 | 3 | 3 |
| LINALOOL | 100 | 100 | 100 | 100 | 100 |
| VERDOX | 20 | 20 | 20 | 20 | 20 |
| VERTENEX | 30 | 30 | 30 | 30 | 30 |
| TERPINEOL | 12 | 12 | 12 | 12 | 12 |
| TERPENYL ACETATE | 25 | 25 | 25 | 25 | 25 |
| *CIS*-3-HEXENOL | 1 | 1 | 1 | 1 | 1 |
| TRIPLAL | 10 | 10 | 10 | 10 | 10 |
| CITRONELLOL | 30 | 30 | 30 | 30 | 30 |
| HEXYLCINNAMIQUE ALDEHYDE | 50 | 50 | 50 | 50 | 50 |
| METHYL DIHYDROJASMONATE | 150 | 150 | 150 | 150 | 150 |
| VANILLINE | 1 | 1 | 1 | 1 | 1 |
| PHENYLETHYL ALCOHOL | 10 | 10 | 10 | 10 | 10 |
| GERANIOL | 30 | 30 | 30 | 30 | 30 |
| ALDEHYDE C14 | 2 | 2 | 2 | 2 | 2 |
| *GAMMA*-DECALACTONE | 1 | 1 | 1 | 1 | 1 |
| BENZYL ACETATE | 10 | 10 | 10 | 10 | 10 |
| IONONE BETA | 10 | 10 | 10 | 10 | 10 |
| FRAMBINONE | 1 | 1 | 1 | 1 | 1 |
| FRUCTONE | 4 | 4 | 4 | 4 | 4 |
| EUCALYPTOL | 1 | 1 | 1 | 1 | 1 |
| DIHYDROFLORIFFONE | 1 | 1 | 1 | 1 | 1 |
| ETHYL MALTOL 5% ALCOOL BENZYLIQUE | 15 | 15 | 15 | 15 | 15 |
| DULCINYL | 5 | 5 | 5 | 5 | 5 |
| DPG | 333 | 313 | 303 | 303 | 303 |
| 2-Ethyl-6,6,6a-trimethyl-hexahydrocyclopenta[b]furan | - | 20 | - | - | - |
| 2-*sec*-Butyl-6,6,6a-trimethyl-hexahydrocyclopenta[b]furan | - | - | 30 | - | - |
| 2-Butyl-6,6,6a-trimethyl-hexahydrocyclopenta[b]furan | - | - | - | 30 | - |
| 2-Isobutyl-6,6,6a-trimethyl-hexahydrocyclopenta[b]furan | - | - | - | - | 30 |

Adding 2-ethyl-6,6,6a-trimethyl-hexahydro-cyclopenta[b]furan to the composition adds lift to the odour as well as power. In addition it gives a fresh and sparkling Bucchu effect, and the berry aspect is enhanced.
Adding 2-*sec*-butyl-6,6,6a-trimethyl-hexahydro-cyclopenta[b]-furan to the composition gives power to the odour and increases the red fruits aspect, it also gives a lift to the lactonic and vanilla notes imparting a "gourmand" aspect.
Adding 2-butyl-6,6,6a-trimethyl-hexahydro-cyclopenta[b]-furan to the composition really increases the red berries aspect, nevertheless with a slight decrease in the odour power. However, the accord 4 has thus a longer lasting note.
Adding 2-isobutyl-6,6,6a-trimethyl-hexahydro-cyclopenta[b]-furan to the composition gives power to the odour and this accord has now a very powerful blackcurrant buds aspect.

## Claims

1. A compound of general formula (I) 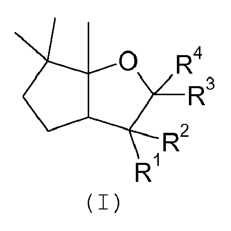 wherein
R¹, R², R³, and R⁴ are, independently, a hydrogen atom or an optionally substituted linear or branched C₁-C₆ alkyl or C₂-C₆ alkenyl group,
provided that when each of R¹, R², and R³ are a hydrogen atom, R⁴ is not a phenyl substituted linear or branched C₁-C₆ alkyl or C₂-C₆ alkenyl group; and
with the proviso that the compound of formula (I) is neither 6,6,6a-trimethyl-hexahydro-cyclopenta[b]furan, 6,6,6a-trimethyl-2-ethyl-hexahydro-cyclopenta[b]furan, nor 6,6,6a-trimethyl-2-propyl-hexahydro-cyclopenta[b]furan.

2. The compound of Claim 1, wherein R¹, R², R³, and R⁴ are, independently, a hydrogen atom or an optionally substituted methyl, ethyl, *n*-propyl, *i*-propyl, *n*-butyl, *i*-butyl, *s*-butyl, *t*-butyl, *n*-pentyl, *i*-pentyl, *t*-amyl, 1-ethyl-propyl, 1,2-dimethyl-propyl, 2,2-dimethyl-propyl, *n*-hexyl, 1-methyl-pentyl group, 2-methyl-pentyl group, 3-methyl-pentyl group, 4-methyl-pentyl group, 1,1-dimethyl-butyl, 1,2-dimethyl-butyl, 1,3-dimethyl-butyl, 2,2-dimethyl-butyl, 3,3-dimethyl-butyl, 1-ethyl-butyl, 2-ethyl-butyl, 1,1,2-trimethyl-propyl, 1,2,2-trimethyl-propyl, 1-ethyl-1-methyl-propyl or 1-ethyl-2-methylpropyl, or an optionally substituted vinyl, 1-propenyl, 1-*i*-propenyl, 2-propenyl, 2-methyl-1-propenyl, 1-butenyl, 2-butenyl, 3-butenyl, 1-pentenyl, 2-pentenyl, 1-hexenyl, 2-hexenyl, 3-hexenyl, 2,4-pentadienyl group, with the proviso that the optional substituent is not a phenyl group.

3. The compound of Claim 1 or 2, wherein R¹, R², and R³, are each a hydrogen atom and R⁴ is an optionally substituted linear or branched C₁-C₆ alkyl or C₂-C₆ alkenyl group, with the proviso that the optional substituent is not a phenyl group.

4. A method of preparing a compound of general formula (I) 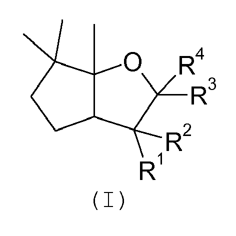 wherein
R¹, R², R³, and R⁴ are, independently, a hydrogen atom or an optionally substituted linear or branched C₁-C₆ alkyl or C₂-C₆ alkenyl group,
the method comprising reacting a compound of formula (III) 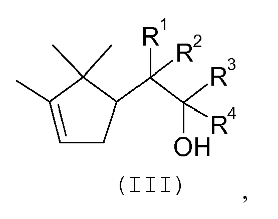 wherein
R¹, R², R³, and R⁴ are defined as in respect of formula (I),
with phosphoric acid in an organic solvent so as to obtain a mixture comprising a compound of formula (I) and a compound of formula (II) 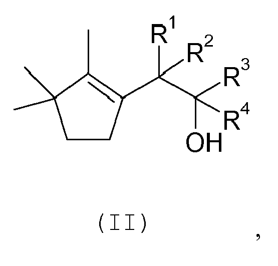 wherein
R¹, R², R³, and R⁴ are defined as in respect of formula (I).

5. The method of Claim 4, wherein the reaction with phosphoric acid is carried out at a temperature of about room temperature to refluxing solvent.

6. The method of Claim 4 or 5, wherein the organic solvent is selected from the group comprising toluene, xylene, trimethylbenzene, cyclohexane, and methylcyclohexane.

7. The method of any of Claims 4 to 6, wherein the phosphoric acid is used in an amount of 1 to 10% by weight of compound (III), preferably 1 to 10% by weight of compound (III).

8. The method of any of Claims 4 to 7, further comprising reacting the compound of formula (II) with trifluoromethanesulphonic acid.

9. The method of Claim 8, wherein the reaction with trifluoromethanesulphonic acid is carried out at a temperature of about room temperature to refluxing solvent, preferably of about 50 to 80°C, even more preferably of about 70 to 80°C.

10. The method of Claim 8 or 9, wherein the trifluoromethanesulphonic acid is used in an amount of about 0.5 to 5% by weight of compound (III), preferably about 0.7 to 3% by weight of compound (III), and even more preferably of about 1 to 2% by weight of compound (III).

11. The method of Claim 8 or 9, comprising, prior to the reaction with trifluoromethanesulphonic acid, the separation and purification of the compound of formula (II).

12. The method of Claim 11, wherein the trifluoromethanesulphonic acid is used in an amount of about 0.5 to 2% by weight of compound (II), preferably about 0.7 to 1.5% by weight of compound (II), and even more preferably of about 1% by weight of compound (II).

13. A compound of general formula (II) 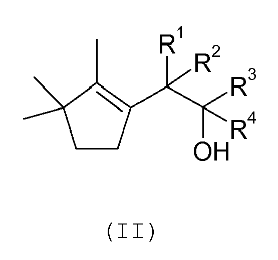 wherein
R¹, R², R³, and R⁴ are, independently, a hydrogen atom or an optionally substituted linear or branched C₁-C₆ alkyl or C₂-C₆ alkenyl group,
provided that when each of R¹, R², and R³ are a hydrogen atom, R⁴ is not a phenyl substituted linear or branched C₁-C₆ alkyl or C₂-C₆ alkenyl group; and
with the proviso that the compound of formula (II) is neither 2-(2,3,3-trimethyl-cyclopent-1-enyl)-ethanol, 1-(2,3,3-trimethyl-cyclopent-1-enyl)-butan-2-ol, nor 1-(2,3,3-trimethyl-cyclopent-1-enyl)-pent-3-en-2-ol.

14. Fragrant and/or flavouring composition containing at least one compound according to any of claims **1** to **3** and **13.**

15. Use of a compound of formula (I) or of formula (II), 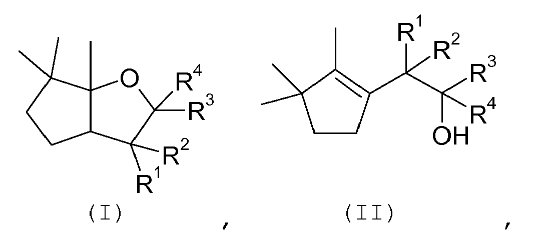 wherein R¹, R², R³, and R⁴ are, independently, a hydrogen atom or an optionally substituted linear or branched C₁-C₆ alkyl or C₂-C₆ alkenyl group, or of a composition according to Claim **14**, in the field of perfumery, for the preparation of perfumed bases and concentrates, fragrances, perfumes; topic compositions; cosmetic compositions, such as face and body creams, cleansers, facial treatment, talc powders, hair oils, shampoos, hair lotions, bath oils and salts, shower and bath gels, soaps, body anti-perspirants and deodorizers, pre-shave, shaving and post-shave creams and lotions, creams, toothpastes, mouth baths, and pomades; and cleaning products, such as softeners, detergents, air deodorizers and household cleaning supplies.

16. Use of a compound of formula (I) or of formula (II), 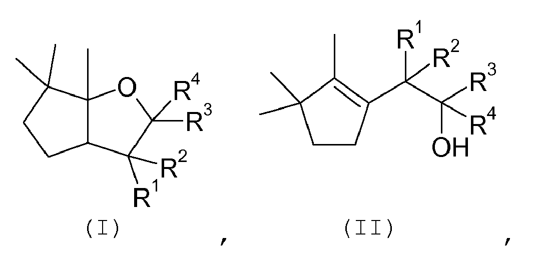 wherein R¹, R², R³, and R⁴ are, independently, a hydrogen atom or an optionally substituted linear or branched C₁-C₆ alkyl or C₂-C₆ alkenyl group, or of a composition according to Claim **14**, as flavouring agent for the preparation of foodstuffs, drinks and tobacco, the foodstuffs and drinks preferably being selected from the group consisting of dairy products, ice creams, soups, sauces, dips, dishes, meat products, culinary assistances, salted biscuits, snacks, soft drinks, beers, wines and spirits.

17. Use of a compound of formula (I) or of formula (II), 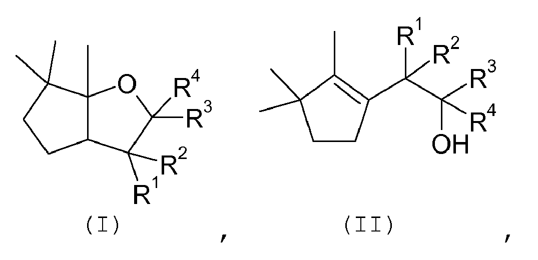 wherein R¹, R², R³, and R⁴ are, independently, a hydrogen atom or an optionally substituted linear or branched C₁-C₆ alkyl or C₂-C₆ alkenyl group, or of a composition according to Claim **14**, as masking agent of odours and/or flavours.

18. Use of a compound of formula (I) according to any of claims **1** to **3** or of a compound of formula (II) according to claim **13**, or of a composition according to Claim **14**, in combination with other perfumed or flavoured ingredients, solvents, additives or fixatives.
